(19) Europäisches Patentamt / European Patent Office / Office européen des brevets

(11) **EP 4 765 155 A1**

(12) **EUROPEAN PATENT APPLICATION**

(43) Date of publication:
**24.06.2026 Bulletin 2026/26**

(21) Application number: **24307243.6**

(22) Date of filing: **20.12.2024**

(51) International Patent Classification (IPC):
**G16H 50/20** (2018.01)    **G16H 50/30** (2018.01)
**G16H 50/70** (2018.01)    **G16H 70/60** (2018.01)

(52) Cooperative Patent Classification (CPC):
**G16H 50/20; G16H 50/30; G16H 50/70;
G16H 70/60**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Université de Franche-Comté
25000 Besançon (FR)**

(72) Inventor: **BREDY-MAUX, Charlotte
Besançon (FR)**

(74) Representative: **Bandpay & Greuter
11, rue Christophe Colomb
75008 Paris (FR)**

(54) **PREDICTING NEONATAL OUTCOMES**

(57) The disclosure notably relates to a computer-implemented method for a method for machine-learning a function. The method is hereinafter referred to as the "machine-learning method", or simply the "method". The function comprises a first model configured for predicting a clinical-biological score representing a health state of a newborn. The clinical-biological score is a function of clinical and biological parameters. The first model takes as input pregnancy data, and optionally fetal heart rate recording data and partogram data. The method comprises obtaining S10 a database of patient deliveries including pregnancy data, fetal heart rate recording data and partogram data and associated clinical-biological scores. The method comprises training 520 the function based on the obtained database. Such a method improves the prediction of neonatal outcomes.

Obtaining a database of patient deliveries — S10

Training the function based on the database — S20

**FIG. 1**

EP 4 765 155 A1

**Description**

**TECHNICAL FIELD**

The disclosure relates to the field of computer programs and systems, and more specifically to a method, system and program for predicting neonatal outcomes.

**BACKGROUND**

When a woman arrives in the delivery room, she undergoes a medical diagnosis that first consists of the admission test. The admission test assesses the mother and her fetus's capacity to undergo a vaginal delivery. Namely, the stage of labor, the maternal temperature and the fetal heart rate are measured. In the case where the admission test is deemed satisfactory, the mother is monitored throughout her labour via the fetal heart rate, her heart rate, and the dynamic of her contractions to detect any anomaly and immediate risks of a vaginal delivery. An analysis of these three constants is then carried out, and completed by second-line clinical and/or biological examinations such as scalp pH to decide the mode of delivery i.e., whether or not an unplanned Caesarean section is necessary or if a vaginal delivery with or without instruments can be conducted. If the admission test is deemed unsatisfactory, a Cesearan section is carried out. After this analysis, it is also necessary to decide whether or not the newborn should be admitted to intensive care. For this, another analysis is carried out based on clinical parameters (e.g., the Apgar score) or biological parameters (such as the cord gasometry). The problem is that there may be a discrepancy between the results provided by the different constants or parameters measured. Another problem is that analyses can sometimes lead to errors.

Within this context, there is still a need for an improved solution for predicting neonatal outcomes and the best mode of delivery at the individual level.

**SUMMARY**

It is therefore provided a method for machine-learning a function. The method is hereinafter referred to as the "machine-learning method", or simply the "method". The function comprises a first model configured for predicting a clinical-biological score representing a health state of a newborn. The clinical-biological score is a function of clinical and biological parameters. The first model takes as input pregnancy data, and optionally fetal heart rate recording data and partogram data. The method comprises obtaining a database of patient deliveries including pregnancy data, fetal heart rate recording data and partogram data and associated clinical-biological scores. The method comprises training the function based on the obtained database.

The method may comprise one or more of the following:

- The clinical-biological score is also a function of the temperature of the newborn;
- The clinical-biological score is equal to a first value indicating that the newborn is well or a second value indicating a complication for the newborn. The clinical-biological score is equal to the second value when:

  ○ an arterial temperature measure is higher than a maximum arterial temperature value, for example between 38 and 39 degrees (e.g. 38.2 degrees);
  ○ an arterial pH measure is lower than a minimum pH measure, for example of 7;
  ○ an arterial lactate measure is higher than a minimum lactate measure, for example of 3 mmol/L;
  ○ a temperature venous measure is higher than a maximum venous temperature value, for example 38; and/or
  ○ an average Apgar score is lower than a maximum Apgar score, for example 8, or

  equal to the first value otherwise.
- The function further comprises a second model configured for predicting an average newborn heart rate in a future period of time. The second model takes as input a history of fetal heart rate recording data;
- The function further comprises a third model configured for predicting a transfer to intensive care or to a Caesarean section. The third model takes as input the clinical-biological score predicted by the first model and the average newborn heart rate predicted by the second model;
- The pregnancy data includes a baby weight, a baby size, a parental socio-professional category, a gestational age and/or data extracted from pregnancy examination reports such as fetal ultrasound biometrics;
- The partogram data includes characteristics of labor, a quality of amniotic fluid, an evolution of cervical dilatation and/or a recording of uterine contractions;
- The function is further configured for outputting an estimate of a 95% confidence interval of the predicted clinical-biological score; and/or

- The first model is further configured to predict:

  ◦ a first value of the clinical-biological score at onset of labor based on pregnancy data, and
  ◦ a second value of the clinical-biological score during labor based on the first value, the fetal heart rate recording data and the partogram data.

It is further provided a method for assessing a health state of a newborn based on the clinical-biological score according to the method of any of the preceding claims. The method is hereinafter referred to as the "assessing method".

The assessing method may comprise one or more of the following:

- performing at least one prediction of the clinical-biological score of the newborn based on the trained function;
- displaying the at least one prediction of the clinical-biological score and/or the predicted average newborn heart rate; and/or
- triggering an alert according to the prediction of the transfer to intensive care or to the Caesarean section.

It is further provided a computer program comprising instructions which, when executed by a computer, cause the computer to carry out the machine-learning method and/or the assessing method.

It is further provided a computer readable storage medium having recorded thereon the computer program.

It is further provided a system comprising a processor coupled to a memory and a graphical user interface, the memory having recorded thereon the computer program.

## BRIEF DESCRIPTION OF THE DRAWINGS

Non-limiting examples will now be described in reference to the accompanying drawings, where:

- FIGs. 1 and 2 show flowcharts of examples of the methods;
- FIG. 3 illustrates an example of the database of patient deliveries;
- FIG. 4 illustrates examples of predictions using the trained function.
- FIGs. 5 to 11 illustrate examples of results; and
- FIG. 12 shows an example of the system.

## DETAILED DESCRIPTION

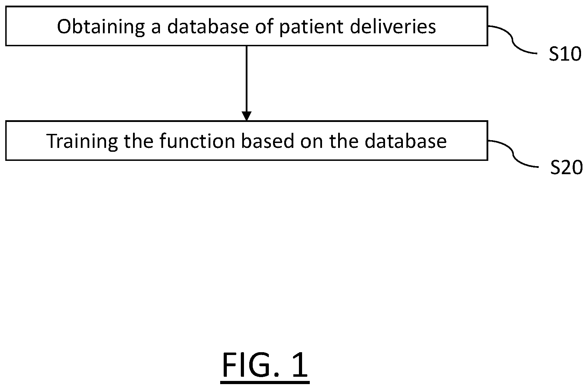

With reference to the flowchart of FIG. 1, it is proposed a computer-implemented method for a method for machine-learning a function. The method is hereinafter referred to as the "machine-learning method", or simply the "method". The function comprises a first model configured for predicting a clinical-biological score representing a health state of a newborn. The clinical-biological score is a function of clinical and biological parameters. The first model takes as input pregnancy data, and optionally fetal heart rate recording data and partogram data. The method comprises obtaining S10 a database of patient deliveries including pregnancy data, fetal heart rate recording data and partogram data and associated clinical-biological scores. The method comprises training S20 the function based on the obtained database.

Such a method improves the prediction of neonatal outcomes and provides a decision tool to assess the safest mode of delivery in real-time, complementary to the fetal heart rate as a stand-alone signal.

Notably, the clinical-biological score improves the assessment of the state of health of a baby, making it particularly relevant for predicting neonatal outcomes. In particular, the clinical-biological score is a function of both clinical parameter(s) and biological parameter(s), which make it more accurate for representing the health state of the newborn as other scores (e.g., the Apgar score). For this, another analysis is carried out based on clinical parameters (e.g., the Apgar score) or biological parameters (such as the cord gasometry). This score solves the problem of discordance between results provided by taking into account only either clinical parameters (e.g. Apgar score), or biological parameters (such as cord gasometry). Moreover, this clinical-biological score is predicted with high accuracy by the trained function. The method therefore improves decision-making concerning neonatal outcomes.

The machine-learning method and the assessing method are computer-implemented. This means that steps (or substantially all the steps) of the methods are executed by at least one computer, or any system alike. Thus, steps of the methods are performed by the computer, possibly fully automatically, or, semiautomatically. In examples, the triggering of at least some of the steps of the methods may be performed through user-computer interaction. The level of user-computer interaction required may depend on the level of automatism foreseen and put in balance with the need to implement user's wishes. In examples, this level may be user-defined and/or pre-defined.

A typical example of computer-implementation of the methods is to perform the methods with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

The dataset considered by the machine-learning method for training the function may be stored in a database. By "database", it is meant any collection of data (i.e. information) organized for search and retrieval (e.g. a relational database, e.g. based on a predetermined structured language, e.g. SQL). When stored on a memory, the database allows a rapid search and retrieval by a computer. Databases are indeed structured to facilitate storage, retrieval, modification, and deletion of data in conjunction with various data-processing operations. The database may consist of a file or set of files that can be broken down into records, each of which consists of one or more fields. Fields are the basic units of data storage. Users may retrieve data primarily through queries. Using keywords and sorting commands, users can rapidly search, rearrange, group, and select the field in many records to retrieve or create reports on particular aggregates of data according to the rules of the database management system being used.

The database comprises real-world data collected from a large number of patients who have delivered (for example over several years for one or more hospitals), ensuring robustness and diversity in the dataset. For each patient, the database contains detailed gestational, socio-demographic as well as delivery-related data that serve as input parameters for the predictive function. At the time of executing of the methods, all of the patient data may already have been measured, and the obtaining S10 of the database may solely comprise retrieving these already measured patient data. Patient data may include pregnancy data (such as maternal age, gestational age, and pregnancy complications), fetal heart rate recording data (capturing metrics like baseline rate, variability and/or decelerations), and partogram data (documenting labor progression, such as cervical dilation and contraction patterns). Additionally, the database includes, for each patient, the corresponding clinical-biological score.

The clinical-biological score associated to each patient included in the database may have been calculated using a formula applied to the data recorded for each patient in the database. The formula may be applied during the executing of the machine-learning method, for example at the obtaining S10 of the database step. In that case, the obtaining S10 may comprise retrieving the database of patient deliveries and computing clinical-biological scores of patients included in the database using the formula. Alternatively, the formula may already have been applied prior to the executing of the machine-learning method. In that case, the obtaining S10 of the database may solely comprise retrieving the database of patient deliveries including the computed clinical-biological scores.

The clinical-biological score is a function of both clinical and biological parameters. It means that the computing of the clinical-biological score takes into account at least one clinical parameter and at least one biological parameter. A biological parameter refers to quantifiable biological data derived from the physiological or biochemical processes of the mother or fetus during labor, such as fetal heart rate, arterial pH, or lactate levels, often obtained through laboratory tests or continuous monitoring equipment. These parameters often require further analysis via laboratory tests or specific sensors, such as arterial pH, lactate levels or blood oxygenation. Examples of biological parameters include an arterial temperature measure, an arterial pH measure, an arterial lactate measure and/or a temperature venous measure (e.g., the clinical-biological score may be function of all of these measures). The clinical-biological score may be computed at a given time, and in that case may consider the values of these parameters at this given time. A clinical parameter refers to measurable data or observations related to a patient's health status during labor and delivery, such as maternal heart rate, uterine contraction frequency, or cervical dilation, typically assessed through clinical monitoring. These parameters may be derived from direct observations or measurements made by medical staff or clinical monitoring devices. These parameters reflect phenomena that are visible or measurable in real time at the macroscopic level. Examples of clinical parameters include the Apgar score. The clinical-biological score may be a function of a clinical parameter derived from one or more Apgar scores measured at different times during delivery (e.g., at 1, 3, 5, 10 minutes), such as an average of these Apgar scores. The clinical-biological score may be equal to a first value indicating that the newborn is well or a second value indicating a complication for the newborn. In examples, the clinical-biological score may incorporate the newborn's temperature as a parameter to enhance accuracy. The inclusion of temperature improves the assessment of the newborn's health state by capturing physiological phenomena that manifest through variations in temperature.

The clinical-biological score may be equal to a first value indicating that the newborn is well or a second value indicating a complication for the newborn. "Indicating the newborn is well" means that the clinical-biological score corresponds to a state where all assessed parameters, such as physiological and biological measures, fall within normal ranges, suggesting that the newborn is healthy and does not require immediate medical intervention. "Indicating a complication for the newborn" means that the clinical-biological score reflects deviations in one or more parameters from their normal ranges, signaling potential issues such as fetal distress, inadequate oxygenation, or other conditions requiring prompt medical attention. These complications may indicate the need for transferring the newborn to an intensive care unit or, if detected during labor, transferring the mother to a facility capable of performing a Caesarean section.

The formula for computing the clinical-biological score may attribute a value based on clinical and biological parameters measured during the delivery. In particular, the formula may attribute a first value indicating that the newborn is well or a second value indicating a complication for the newborn. The formula may attribute the second value when:

- an arterial temperature measure is higher than a maximum arterial temperature value, for example, between 38°C and 39°C (e.g., 38.2°C);
- an arterial pH measure is lower than a minimum pH measure, for example, 7;
- an arterial lactate measure is higher than a minimum lactate measure, for example, 3 mmol/L;
- a venous temperature measure is higher than a maximum venous temperature value, for example, 38°C; and/or
- an average Apgar score is lower than a maximum Apgar score, for example, 8.

Otherwise, the formula may attribute the first value. For example, the formula may determine whether the arterial temperature measurement, the arterial pH measurement, the arterial lactate measurement, the venous temperature measurement or the average Apgar score are within the predetermined ranges indicated above. When at least one value is not within the given predetermined range (e.g., two or more of the values are not within their respective ranges), the formula may attribute the second value. Alternatively, when all values are within their respective ranges, the formula may attribute the first value.

The dataset may be stored in a database. The obtaining S10 of the dataset may comprise retrieving the dataset from the database. Then, the obtaining of the dataset may comprise storing the retrieved dataset in memory. After the recording, the machine-learning method may perform the training S20 of the function based on the recorded dataset. Alternatively, the obtaining S10 of the dataset may comprise providing an access to the dataset in the database. In that case, the machine-learning method may use this access to perform the training S20 of the function.

The training S20 of the function may be performed in any manner. The training S20 of the function may comprise the training of each model (i.e., the first, second, and/or third models) that the function includes. The training S20 of the function may comprise training the first model to predict the clinical-biological scores recorded for each patient in the database based on the patient data recorded in the database. The training S20 of the function may comprise training the second model to predict average newborn heart rates of patients based on the fetal heart rate recording data recorded for each patient in the database. The training S20 of the function may comprise training the third model to predict neonatal outcomes (including, e.g., transfer to intensive care or to a Caesarean section) based on the patient data recorded in the database. Each model may be a machine-learning model providing predictions based on a respective set of weights.

The training S20 of the function may include dividing the database into a training set and a test set (e.g., 70% for training and 30% for test). The training set is used to fit each model by exposing it to a portion of the dataset to learn patterns and relationships between input parameters and the target outcomes. The test set, which consists of the remaining portion of the database, is reserved for evaluating the performance of each model after training, ensuring the generalizability and accuracy of predictions on new, unseen data.

The models used for training S20 may include any type of predictive model suitable for the task. These may include traditional machine learning models such as decision trees, random forests, and support vector machines (SVM), as well as more advanced neural network architectures, such as artificial neural networks (ANN), convolutional neural networks (CNN), and recurrent neural networks (RNN).

During training S20, the respective set of weights for each model may be determined by minimizing an objective function that penalizes the difference between the predicted values and the true values stored in the database. Optimization techniques such as gradient descent or backpropagation may be employed, with hyperparameter tuning and cross-validation used to further enhance performance. By combining these steps, the training ensures each model can reliably predict clinical-biological scores, average newborn heart rates, and neonatal outcomes based on the data available.

The trained function includes a first model configured to predict a clinical-biological score that represents the health state of a newborn. The first model takes as input pregnancy data, and optionally, fetal heart rate recording data and partogram data. The pregnancy data may include factors such as maternal age, gestational age, biometrics data collected by fetal scan throughout the three trimesters of pregnancy and any complications observed during pregnancy, which are critical for assessing the newborn's anticipated health. For example, the pregnancy data may include a baby weight, a baby size, a parental socio-professional category, a gestational age and/or data extracted from pregnancy examination reports such as fetal ultrasound biometrics. The fetal heart rate recording data may include baseline heart rate values, variability, and accelerations or decelerations observed at specific intervals, providing insight into fetal well-being. The partogram data may include information documenting the progression of labor, such as cervical dilation, contraction patterns, and fetal station, which are key indicators of labor progress and potential complications. For example, the partogram data may include characteristics of labor, a quality of amniotic fluid, an evolution of cervical dilatation and/or a recording of uterine contractions. Based on these inputs, the first model outputs a prediction of the clinical-biological score, which is a function of the provided data and helps assess the newborn's health state at the onset of labor.

In examples, the function further may comprise a second model configured to predict an average newborn heart rate in a future period of time. The second model takes as input a history of fetal heart rate recording data, which may include measurements such as baseline heart rate, variability, and episodes of accelerations or decelerations observed during labor. This historical data provides important insights into fetal well-being and helps anticipate the newborn's heart rate after delivery. By analyzing trends and patterns in the fetal heart rate data over time, the second model predicts the average heart rate for the newborn, offering an indication of potential health issues such as fetal distress or other complications. This prediction aids in making informed decisions regarding the need for medical intervention during or after the delivery process.

In examples, the function further comprises a third model configured to predict the need for a transfer to intensive care or a Caesarean section. The third model takes as input the clinical-biological score predicted by the first model and the average newborn heart rate predicted by the second model. By analyzing these inputs, the third model assesses the likelihood of complications that may require urgent medical intervention, such as the need for the newborn to be transferred to an intensive care unit or the need for a Caesarean section during delivery. The clinical-biological score provides a comprehensive evaluation of the newborn's health state, while the predicted average heart rate offers additional insight into fetal well-being. Together, these factors enable the third model to generate a prediction that helps guide medical decision-making, ensuring timely and appropriate care during delivery.

In examples, the function is further configured for outputting an estimate of a 95% confidence interval of the predicted clinical-biological score. For example, let P be the individual prediction of a transfer to the neonate ICU (resp. of a clinical-biological score equal to 0). Let s be the standard deviation of the predictions over the training (resp. testing) sets of size $n_{train}$ (resp. $n_{test}$). The sample size is denoted n without loss of generality in the formula. Based on the data, the model predicts a transfer (resp. a clinical-biological score greater than 1) if the probability of being transferred (resp. of a clinical-biological score greater than 1) exceeds 50%. For every individual in the database, the model thus yields a probability for each output i.e., the continuous version of the prediction, and a binary value equal to 1 or 0 i.e., the discrete version of the prediction, depending on whether the probability is greater or smaller than 50%. Thus, the confidence interval gives the interval such that there is 95% chance that the true probability of being transferred (resp. of a clinical-biological score equal to 0) belongs to it. For example, the model might predict a 72% probability of being transferred for a given neonate and will thus indicate "transfer = 1". But the 95% confidence interval of this probability is [69%;75%]. This information is particularly beneficial for assessing cases of probability close to 50%. The function may for example output an estimate of the 95% confidence interval of the predicted clinical-biological score using the following formula:

$$IC = \left[P - 1.96 * \left(\frac{s}{\sqrt{n}}\right); P + 1.96 * \left(\frac{s}{\sqrt{n}}\right)\right]$$

wherein *IC* is the confidence interval estimated, P is the individual prediction of a transfer to the neonate ICU, s is the standard deviation of the predictions over the training and *n* is the sample size. 1.96 is the quantile of order 0.95 such that there is a 95% chance that the true value of the risk prediction lies within this interval.

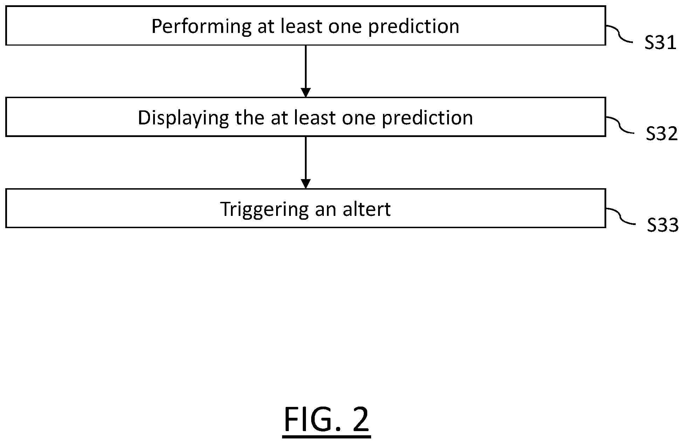

With reference to FIG. 2, the assessing method is now discussed. The assessing method is for assessing a health state of a newborn during labor and delivery. Assessing the health state refers to providing a quantitative indication of the newborn's condition through a predicted clinical-biological score. This clinical-biological score is a calculated value that reflects various physiological and biological factors associated with the newborn's health. The score serves as an informative tool for medical personnel, enabling them to make well-informed decisions. The assessing method itself, however, does not include any steps of decision-making or parameter acquisition, focusing strictly on computation and display.

In examples, the assessing method may comprise directly computing the clinical-biological score based on pre-recorded parameters, which are physiological or biological measurements performed during labor. These parameters are stored in memory before the execution of the assessing method. In other implementations, the assessing method may comprise performing S31 at least one prediction of the clinical-biological score based on the trained function, that processes the pre-recorded parameters to generate the at least one prediction. The parameters used as inputs include measurements relevant to the newborn's condition, such as arterial temperature, arterial pH, arterial lactate concentration, venous temperature, or the average Apgar score.

The predictions generated during the assessing method may include a first prediction of the clinical-biological score at the onset of labor, which is computed solely on pregnancy data. This means that the first model may use only pregnancy data as input for this initial prediction. This first prediction reflects maternal and prenatal factors, such as gestational age, maternal health status, and any complications observed during pregnancy, all of which are relevant to the newborn's anticipated health state at the start of labor. As labor progresses, a second prediction of the clinical-biological score may be computed, which updates the initial score by incorporating additional data collected during labor. This second prediction takes into account the first prediction of value and includes fetal heart rate recording data, which provides critical information about fetal well-being during labor, as well as partogram data, which documents the progression of labor, such as cervical dilation and contraction patterns. For this second prediction, the first model may use the first predicted value, along with fetal heart rate recording data and partogram data measured during labor, to output an updated second value of the clinical-biological score. This second prediction may be the same as the first prediction or different if the fetal heart rate recording data and partogram data indicate a potential complication for the newborn. By combining both prenatal and intrapartum data, the assessing method enables dynamic and more accurate prediction of the newborn's clinical-biological score throughout the delivery process.

After the clinical-biological score is calculated or predicted S31, the assessing method may comprise displaying S32 it in a format visible to medical personnel, such as doctors or nurses, allowing them to review the information during delivery. This display step ensures the accessibility of the clinical-biological score for medical interpretation without intervening in the clinical workflow. The assessing method does not encompass parameter measurement or any steps of decision-making, focusing solely on the computation and presentation of the clinical-biological score.

The assessing method may further comprise additional steps to enhance its applicability in clinical settings by ensuring the effective communication of predictions and the prompt identification of critical situations. One of these steps comprises displaying S32 at least one of the following predictions: the clinical-biological score, which quantifies the newborn's health state, and/or the predicted average newborn heart rate, which provides insight into the newborn's cardiac status. These predictions are shown on an interface accessible to medical personnel, such as a monitor or a handheld device. The display is designed to ensure clarity and immediate visibility during labor and delivery, enabling healthcare professionals to quickly review and interpret the information.

Another step comprises triggering S33 an alert based on the generated predictions when critical conditions are identified. For instance, an alert may be issued if the predicted clinical-biological score or related parameters indicate a need for the newborn's transfer to intensive care. Similarly, an alert can be triggered if the predictions suggest the necessity of an emergency Caesarean section due to fetal distress or complications during delivery. The alert system may include visual signals (e.g., flashing icons) or auditory signals (e.g., alarms), ensuring that high-risk situations are promptly flagged. These steps support timely clinical intervention while maintaining the decision-making authority of medical personnel.

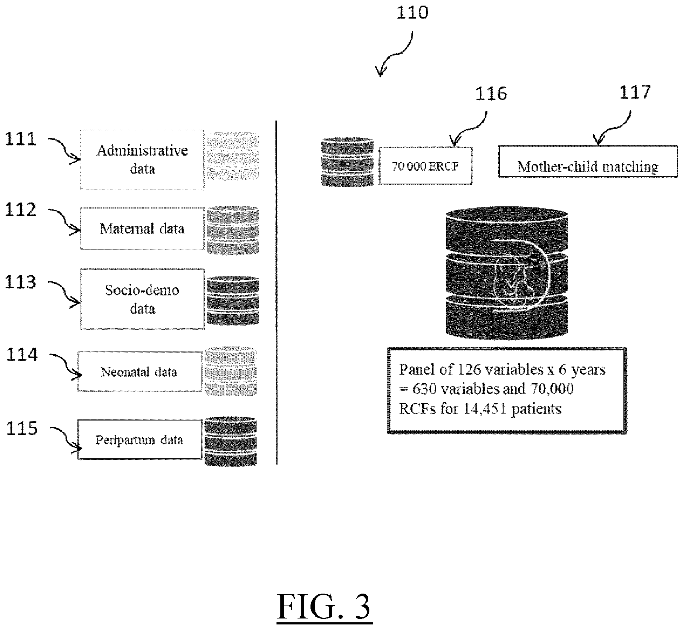

With reference to FIGs. 3 to 12, examples of implementations of the methods are now discussed.

Every year, neonatal complications affect a significant percentage of births. These complications can lead to transfers to the intensive care unit (ICU), avoidable or unplanned caesarean sections, which have a high iatrogenic, logistical, organizational, and financial impact on healthcare teams, families, and hospitals. Moreover, doctors need to anticipate and react quickly to health risks for newborns, particularly in the event of hypoxia or neonatal distress. However, current assessment criteria, such as fetal heart rate (ERCF) curves, remain subjective and insufficiently predictive. Within this context, there is still a need for an improved solution for predicting neonatal outcomes.

There is a lack of objective and reliable tools to predict early clinical-biological outcomes (e.g., pH, lactates, Apgar score) and the necessity for transfers to neonatal care units. Additionally, there is variability between clinicians in interpreting obstetrical and neonatal data, which can lead to suboptimal decisions. Iatrogenic sequelae are also associated with peripartum and postnatal interventions in case of complications for patients (e.g., multi-scarred uterus in the case of avoidable caesarean sections or perineal tears in the case of unplanned caesareans) and for newborns (e.g., hypoxia and transfers).

In this context, it is provided a predictive system based on artificial intelligence (AI), which is based on a function trained using a large medical database (including 70,000 ERCFs) to predict neonatal outcomes and ERCF data. In particular, the system includes the modeling of a clinical-biological score that surpasses the predictive benchmark of the Apgar score. This score integrates venous-arterial pH, venous-arterial lactates, neonatal temperature, and Apgar scores to assess newborn well-being. The use of AI also helps reduce subjectivity in the interpretation of obstetric data, ensuring more accurate and consistent predictions.

The primary objective of this approach is to predict the neonatal outcome at an individual level, considering the clinical-biological context. A secondary objective is to estimate the uncertainty associated with each prediction, ensuring an agnostic approach with a guarantee of coverage. A tertiary objective is to interpret the model and its outputs, focusing on medico-legal ethics, safety, and interoperability.

The implementation of this predictive function brings several significant impacts. Firstly, it improves healthcare by enabling a more precise anticipation of neonatal complications, leading to faster and more appropriate management of the newborn's health. Secondly, it optimizes healthcare efficiency by reducing unnecessary transfers to intensive care units and enabling more efficient resource management within hospitals. Finally, the function provides added value to partner university hospitals (CHUs) by integrating data sharing into the daily practice of medical teams, thereby enhancing decision-making processes and supporting clinical outcomes. By incorporating these elements, the predictive function aims to enhance the quality and efficiency of neonatal care, reduce unnecessary interventions, and contribute to better outcomes for both mothers and newborns.

The training of the function involves the creation of a custom database of patient deliveries that consolidates data from the start to the end of pregnancy, neonatal transfers, and fetal heart rate (ERCF) recordings for 12,000 patients and 16,000 births at the CHU Jean Minjoz in Besançon between 2017 and 2022. FIG. 3 illustrates an example of database 110 of patient deliveries. This comprehensive database serves as the foundation for several predictive models designed to enhance neonatal care. Additionally, the function trained for predicting a new clinical-biological score that takes into account clinical and biological parameters.

Decisions regarding neonatal transfer to intensive care may rely independently on the Apgar Score (clinical) and cord blood gas analysis (biological). However, these two factors often provide discordant results when interpreted separately, with a normal gas analysis but concerning clinical signs, or vice versa. The clinical-biological score predicted by the function integrates these elements along with the newborn's temperature, providing a more accurate and reliable tool for decision-making and better predicting neonatal outcomes. It is a significantly better predictor of neonatal outcome than Apgar Score or gasometry.

The database 110 used for training the function predicting the said clinical-biological score may gather several types of data. In particular, for each patient, the database 110 may include the following data. The database may include administrative data 111 (e.g., 6 variables). The database may include maternal data. Maternal data may include 10 variables, for example representing maternal weight at start of pregnancy, maternal height, BMI in early pregnancy, weight gain and BMI end of pregnancy, Gestate, parity and/or type of pregnancy (e.g., single, twin, triple). The database may include socio-demo data (e.g., 3 variables). The database may include neonatal data. Neonatal data may include 15 variables, for example representing newborn condition, gender, birth weight, birth size, PC, BIP, PT, Apgar score (e.g., at 1, 3, 5, 10 minutes), pH arterial, venous pH, lactate arterial, resuscitation at birth, newborn discharged from delivery room and/or place of transfer. The database may include peripartum data. Peripartum data may include 92 variables, for example representing labor characteristics, delivery characteristics and/or ultrasound data. In all, the database may include a panel of 126 variables over 6 years, i.e. 630 variables, for 14,451 patients.

The database may also include 70,000 Electronic Records of Fetal Heart (ERCF) of patients 116. ERCF are digital files that contain detailed information about the fetal heart rate (FHR) and other related parameters collected during pregnancy and labor. These records may include continuous FHR tracings, which monitor the rhythm and rate of the fetal heart, along with other relevant data such as accelerations, decelerations, and variability in the heart rate. ERCF may include data points recorded at specific time intervals or frequencies. These data points are collected at regular intervals to provide continuous monitoring of the fetal heart rate and other relevant parameters. Additionally, ERCF may capture other variables such as uterine contractions, maternal heart rate, and oxygenation levels, providing a comprehensive view of fetal well-being. This data is typically recorded using electronic fetal monitoring systems, which track the fetus's vital signs over time and help healthcare providers assess potential risks during labor and delivery. The construction of the database may include performing a mother-child to match newborn and mother data.

Another feature is the prediction of baseline fetal heart rate using the second model. Especially, the second model is trained, using the ERCF data recorded in the database of patient deliveries, for predicting an average newborn heart rate in a future period of time based on history of fetal heart rate recording data.

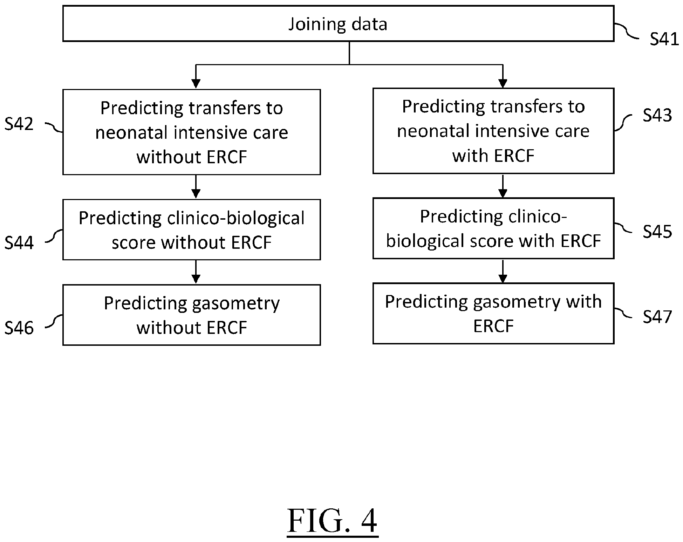

As illustrated in FIG. 4, three predictive protocol arms may be used based on the data to predict both the clinical-biological score and the need for neonatal transfer to intensive care. The obtaining of the function may include a first step of joining S41 the data, including clinical and biological aspects from pregnancy to delivery, newborn transfer clinics, and tococardiographic (ERCF). Then, the function may be trained following the three predictive protocol arms. In particular, the function may be trained to perform: (i) pre-admission prediction (first prediction), (ii) prediction updated during labor (second prediction), and (iii) an estimation of a 95% confidence interval to reflect uncertainty (estimation of confidence interval). An AI system (Arm A) predicts the outcomes separately for the clinical-biological score S44 and neonatal transfer S42, using all pregnancy data but excluding ERCF data. Arm B uses only the ERCF data to predict the outcomes, while Arm C incorporates both pregnancy and ERCF data for predicting the clinical-biological score S45 and neonatal transfer S43. In example, the function may also be trained for predicting gasometry without ERCF data S46 and with ERCF data S46. By comparing the performance of these three arms, the value of including ERCF data in predicting neonatal outcomes may be assessed.

The clinical-biological score and its predictions set a new benchmark in managing newborns, both for those requiring intensive care and those who do not. This new approach provides a more accurate means of assessing neonatal health and improving decision-making. The database also links clinical, biological, tococardiographic, ultrasound, and neonatal and maternal outcomes. This data is invaluable for conducting various studies and answering critical questions in obstetrics, enhancing research and clinical practices.

Moreover, the integration of the clinical-biological score with AI improves patient and newborn management by offering more precise predictions. The method also simplifies the interpretation of fetal heart rate patterns, reducing errors in analyzing decelerations and accelerations, which currently lack consensus in clinical practice. This advancement ensures better fetal monitoring, contributing to more reliable assessments and timely interventions.

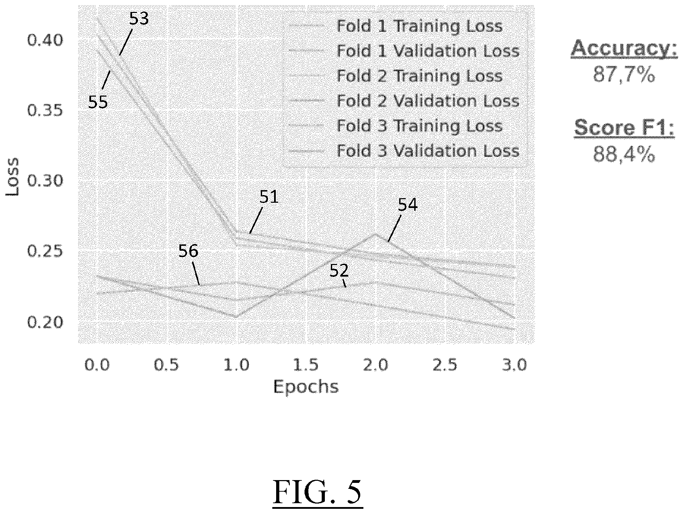

FIGs. 5 to 11 illustrate examples of results obtained by the trained function during the assessing method. Performance evaluation metrics may include an accuracy, which may represent rate of correct predictions on all individuals. The accuracy may be evaluated using the following formula:

$$\frac{TP + TN}{TP + TN + FP + FN}$$

wherein TP is true positive, TN is true negative, FP is false positive, and FN is false negative.

Performance evaluation metrics may also include an F1-score, which may be computed using the following formula:

$$\frac{TP}{TP + \frac{1}{2}(FN + FP)}$$

Performance evaluation metrics may also include an ROC-AUC sensitivity/specificity trade-off value: when one of TP or TN increases, the other mechanically decreases.

Table 1 below shows the results of predictions of clinical-biological scores ("BM score") using the trained function for several years, as well as predictions of "Apgar scores". The figure shows that the predictions of clinical-biological scores are more accurate than those of Apgar scores (higher accuracy).

Table 1

| | BM Score 2022 | Apgar Score 2022 | BM Score 2021 | Apgar Score 2021 | BM Score 2020 | Apgar Score 2020 | BM Score 2019 | Apgar Score 2019 |
|---|---|---|---|---|---|---|---|---|
| **Sample size** | 2804 | 2804 | 2962 | 2962 | 2741 | 2741 | 2655 | 2655 |
| **AIC** | 2980.5 | 2872.8 | 2821.6 | 2803.3 | 2902.8 | 2779.0 | 2822.4 | 2853.1 |
| **Accuracy Training Set** | 78.88% | 79.32% | 78.20% | 80.09% | 77.84% | 81.19% | 77.81% | 79.18% |
| **Accuracy Testing Set** | 79.02% | 77.14% | 78.44% | 77.64% | 78.00% | 77.01% | 77.93% | 75.13% |

FIG. 5 illustrates results of the training and validation loss functions of the third model predicting neonatal resuscitation transfers without ERCF data after selection of variables by Tree of Decision Tree after a 5-fold cross-validation. Especially, the curves show the loss evolution over epochs: fold 1 training loss (curve 51) and validation loss (curve 52), fold 2 training loss (curve 53) and validation loss (curve 54), and fold 3 training loss (curve 55) and validation loss (curve 56). The goal of this figure is to detect potential over-fitting. Indeed, over-fitting is translated by loss functions fitted on the training sets systematically lower than loss functions fitted on the validation sets and/or when the loss functions diverge. Here, the loss functions converge towards a [0.20;0.25] bandwidth and the validation loss functions sit under the training loss functions. Hence, there is no pathological signs of over-fitting based on the loss functions analysis.

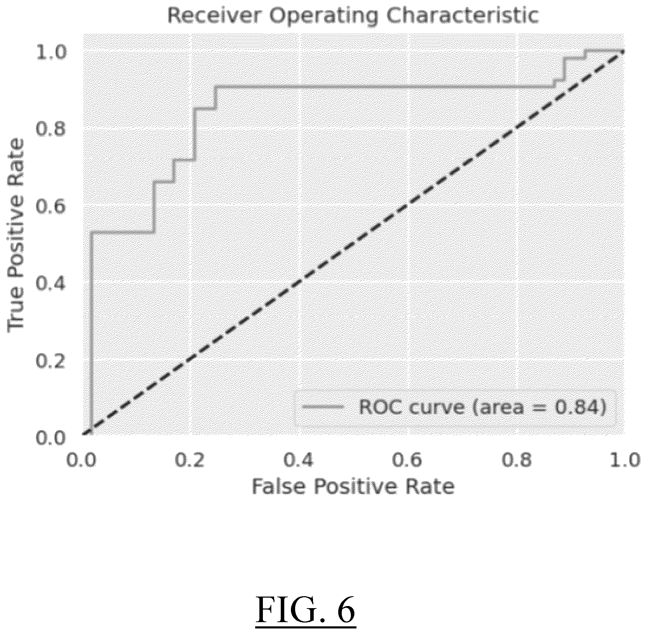

FIG. 6 illustrates the ROC-AUC metric of the third model predicting neonatal resuscitation transfers without ERCF data after selection of variables by Tree of Decision Tree. The ROC-AUC indicates the ratio of true positives over false positives. Here, a false positive corresponds to the model predicting that a newborn will be transferred i.e., yielding the value 1, when the said newborn will in fact not be transferred i.e., yielding the value 0. A true positive corresponds to the case where a prediction of transfer matches with an observed transfer. A model with a ROC-AUC of 0.5 is called a naive model, and predicts a transfer with a constant probability of 50%. A robust benchmark in Deep Learning is a ROC-AUC of 90%.

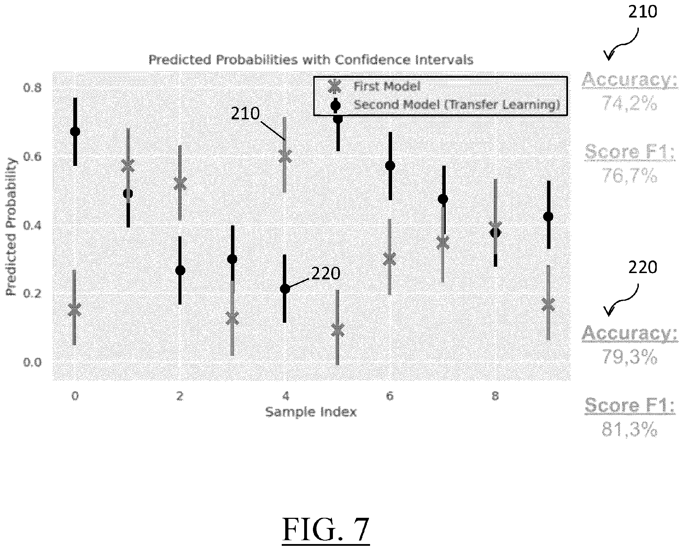

FIG. 7 illustrates the uncertainty estimation i.e., the 95% confidence interval at the individual level updated during labor for 10 newborns randomly sampled from the test set. The prediction 210 is the one before the moment of the admission test, and the prediction 220 is the one updated during labor. Taking the first randomly selected newborn of index 0 at the left side of the figure, the prediction of transfer 210 is about 18-19% i.e., the average probability of transfer for that particular newborn. The 95% confidence interval for that average probability prediction is [0.05;0.25], meaning that there is a 95% chance that the true probability of transfer for that particular newborn is between 5% and 25%.

The 220 value for that same newborn is about 67% with a 95% confidence interval equal to [0.59;0.79].

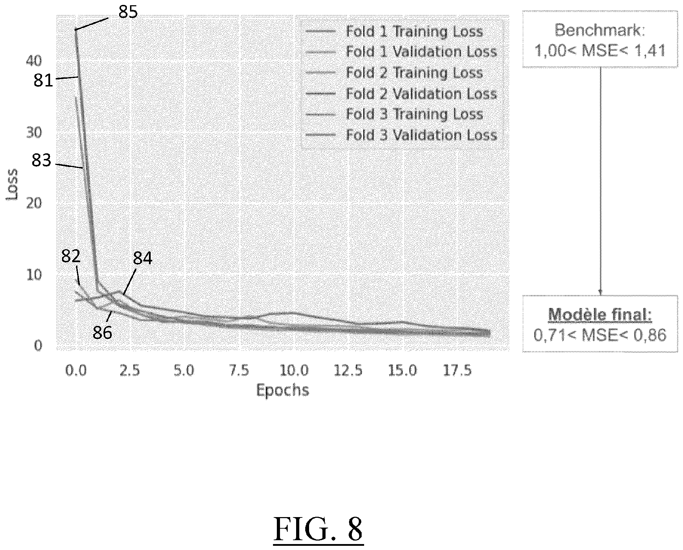

FIG. 8 illustrates the model's training and validation loss functions after 3-fold cross-validation predicting the clinical-biological score without ERCF data on all variables. Especially, the curves show the loss evolution over epochs: fold 1 training loss (curve 81) and validation loss (curve 82), fold 2 training loss (curve 83) and validation loss (curve 84), and fold 3 training loss (curve 85) and validation loss (curve 86). As shown in FIG. 5, the loss functions converge and do not show any pathological signs of over-fitting.

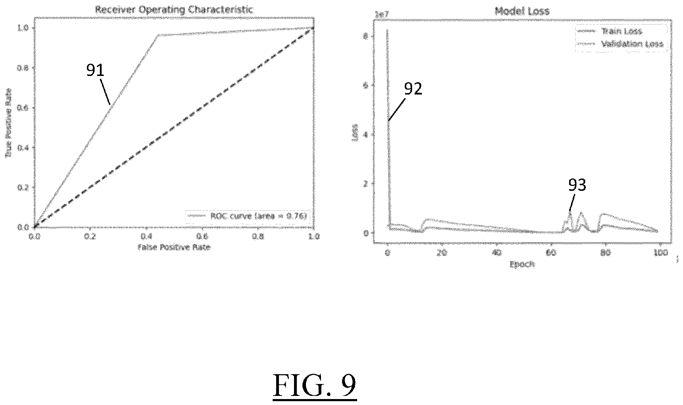

FIG. 9 illustrates the ROC-AUC 91 of the first model predicting the clinical-biological scores and the training 92 and validation 93 loss functions. Especially, the figures show the resulting performance metrics when the first model does and does not take ERCF values into account.

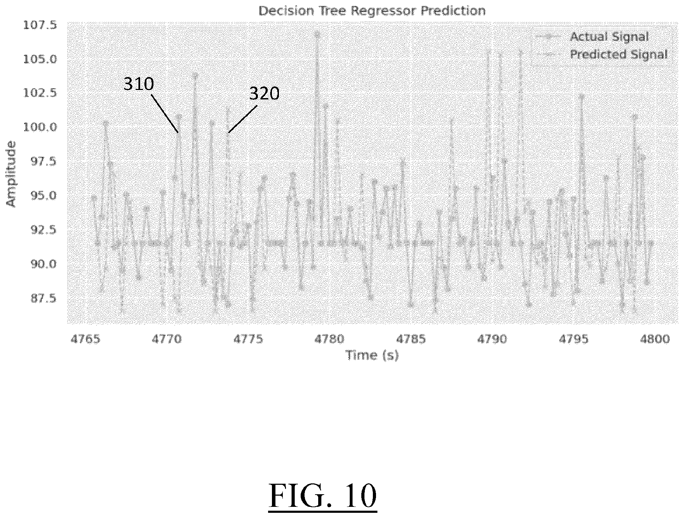

FIG. 10 illustrates the prediction of the second model configured for predicting an average newborn heart rate in a future period of time. The figure shows an example of the history of fetal heart rate recording data 310 taken as input by the second model. The figure also shows the newborn heart rate predicted 320.

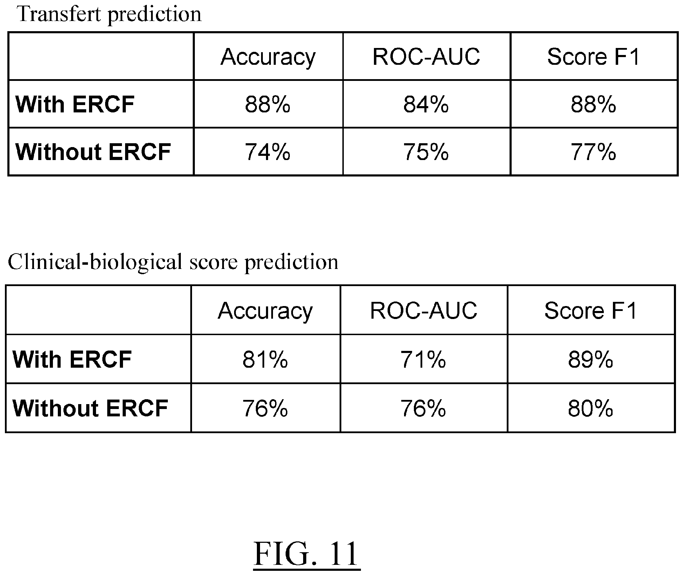

FIG. 11 presents tables summarizing the performance metrics achieved by the trained function, particularly for predicting transfers to intensive care or Caesarean sections using the third model, and for predicting clinical-biological scores using the first model. The results in the tables demonstrate that the trained function delivers accurate predictions for both outcomes. Indeed, the accuracy metric is the easiest to interpret and the less specific. Accuracy computes the total of correct predictions (true positives and true negatives) over all predictions made (true positives, true negatives, false positives and false negatives). The ROC-AUC accounts specifically for the ratio of true positives over false positives and the F1-Score computes the harmonic mean of precision and recall, more precisely it computes a ratio between true positives, and false negatives and false positives.

The architecture of the first model is now discussed. The first model may comprise an architecture of a sequential model, which includes symmetry, robustness and balance of performance metrics. The architecture may comprise the following successive layers:

- an input layer,
- a layer of 64 neurons (powers of 2 and 8, much more stable) with a 'relu' activation function, and L2 regularization of 0.01 (128 parameters),
- a dropout layer of 0.5,
- a layer of 32 neurons (2080 parameters),
- a dropout layer of 0.5, and
- an output layer with a sigmoid activation function (33 parameters).

Optimization may be performed by Adam method, on the binary cross-entropy loss function, over 100 iterations per batch of 32 inputs (total: 2241 parameters - 8.75 kB). Downstream, the model may include an estimation of prediction uncertainty by 95% confidence intervals at individual and population levels.

The architecture of the third model is now discussed. The third model may comprise an architecture of a sequential model with transfer learning, which includes symmetry, robustness and balance of performance metrics. The architecture may comprise the following two models:

The first models computing baseline predictions of the clinical-biological score and of transfer the neonate intensive care unit respectively include the following layer:

- An input layer,
- A layer of 20 neurons (powers of 2 and 8, much more stable) with a 'selu' activation function,
- A batch normalization layer,
- A layer of 5 neurons with a 'selu' activation function,
- A layer of 5 neurons with a 'selu' activation function,
- A layer of 5 neurons with a 'selu' activation function, and
- An output layer with a sigmoid activation function (33 parameters) with a personal loss function.

The models computing the updated predictions of the clinical-biological score and of transfers to the neonate intensive care unit includes the following layer:

- An input layer taking as input the output of the initial model and new data (ERCF, partogram), and
- An output layer with personal loss function.

The model predicting the fetal heart rate include a long-term short term architecture.

Downstream, the model may also include an estimation of prediction uncertainty by 95% confidence intervals at individual and population levels.

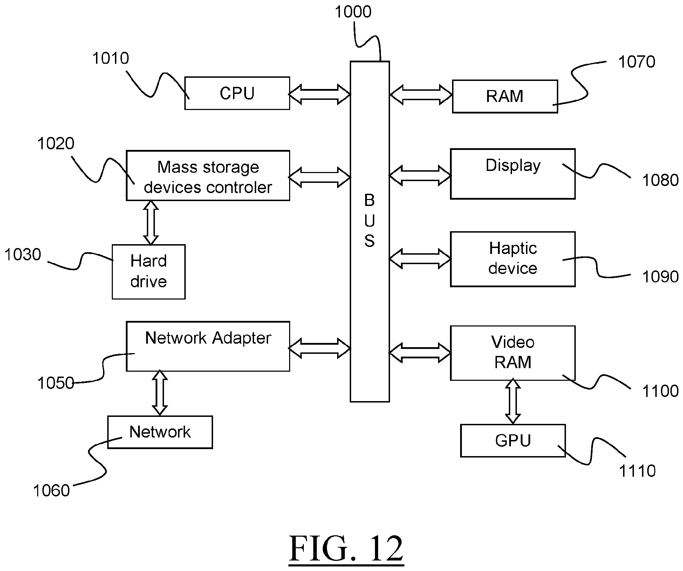

FIG. 12 shows an example of the system, wherein the system is a client computer system, *e.g.* a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

**Claims**

1. A computer-implemented method for machine-learning a function, the function comprising a first model configured for predicting a clinical-biological score representing a health state of a newborn, the clinical-biological score being a function of clinical and biological parameters, the first model taking as input pregnancy data, and optionally fetal heart rate recording data and partogram data, the method comprising:

   • obtaining (S10) a database of patient deliveries including pregnancy data, fetal heart rate recording data and partogram data and associated clinical-biological scores; and
   • training (S20) the function based on the obtained database.

2. The method of claim 1, wherein the clinical-biological score is also a function of the temperature of the newborn.

3. The method of claim 1 or 2, wherein the clinical-biological score is equal to a first value indicating that the newborn is well or a second value indicating a complication for the newborn, the clinical-biological score being:

   • equal to the second value when:

      ○ an arterial temperature measure is higher than a maximum arterial temperature value, for example between 38 and 39 degrees (e.g. 38.2 degrees);
      ○ an arterial pH measure is lower than a minimum pH measure, for example of 7;
      ○ an arterial lactate measure is higher than a minimum lactate measure, for example of 3 mmol/L;
      ○ a temperature venous measure is higher than a maximum venous temperature value, for example 38 ; and/or
      ○ an average Apgar score is lower than a maximum Apgar score, for example 8, or

   • equal to the first value otherwise.

4. The method of any one of claims 1 to 3, wherein the function further comprises a second model configured for predicting an average newborn heart rate in a future period of time, the second model taking as input a history of fetal heart rate recording data.

5. The method of claim 4, wherein the function further comprises a third model configured for predicting a transfer to intensive care or to a Caesarean section, the third model taking as input the clinical-biological score predicted by the first model and the average newborn heart rate predicted by the second model.

6. The method of any one of claims 1 to 5, wherein the pregnancy data includes a baby weight, a baby size, a parental socio-professional category, a gestational age and/or data extracted from pregnancy examination reports such as fetal ultrasound biometrics.

7. The method of any one of claims 1 to 6, wherein the partogram data includes characteristics of labor, a quality of amniotic fluid, an evolution of cervical dilatation and/or a recording of uterine contractions.

8. The method of any one of claims 1 to 7, wherein the function is further configured for outputting an estimate of a 95% confidence interval of the predicted clinical-biological score.

9. The method of any one of claims 1 to 8, wherein the first model is further configured to predict a first value of the clinical-biological score at onset of labor based on pregnancy data, and a second value of the clinical-biological score during labor based on the first value, the fetal heart rate recording data and the partogram data.

10. A method for assessing a health state of a newborn based on the clinical-biological score according to the method of any of the preceding claims.

11. The method of claim 10, comprising:

    • performing at least one prediction of the clinical-biological score of the newborn based on the trained function.

12. The method of claim 10 or 11, further comprising:

    • displaying the at least one prediction of the clinical-biological score and/or the predicted average newborn heart rate; and/or
    • triggering an alert according to the prediction of the transfer to intensive care or to the Caesarean section.

13. A computer program comprising instructions which, when executed by a computer, cause the computer to carry out the method of any of claims 1 to 9 and/or the method of any of claims 10 to 12.

14. A computer readable storage medium having recorded thereon a computer program of claim 13.

15. A system comprising a processor coupled to a memory, the memory having recorded thereon the computer program of claim 1.

Obtaining a database of patient deliveries — S10

Training the function based on the database — S20

# FIG. 1

| Performing at least one prediction | S31 |

↓

| Displaying the at least one prediction | S32 |

↓

| Triggering an altert | S33 |

# FIG. 2

110

116    117

111    Administrative
       data

112    Maternal data

113    Socio-demo
       data

114    Neonatal data

115    Peripartum data

70 000 ERCF    Mother-child matching

Panel of 126 variables x 6 years
= 630 variables and 70,000
RCFs for 14,451 patients

FIG. 3

```
┌─────────────────────────────────────────────────────────────┐
│                        Joining data                          │ ⌐ S41
└─────────────────────────────────────────────────────────────┘
```

| S42 | Predicting transfers to neonatal intensive care without ERCF | Predicting transfers to neonatal intensive care with ERCF | S43 |
| S44 | Predicting clinico-biological score without ERCF | Predicting clinico-biological score with ERCF | S45 |
| S46 | Predicting gasometry without ERCF | Predicting gasometry with ERCF | S47 |

# FIG. 4

Fold 1 Training Loss
Fold 1 Validation Loss
Fold 2 Training Loss
Fold 2 Validation Loss
Fold 3 Training Loss
Fold 3 Validation Loss

Accuracy:
87,7%

Score F1:
88,4%

FIG. 5

FIG. 6

FIG. 7

FIG. 8

FIG. 9

FIG. 10

Transfert prediction

|  | Accuracy | ROC-AUC | Score F1 |
|---|---|---|---|
| **With ERCF** | 88% | 84% | 88% |
| **Without ERCF** | 74% | 75% | 77% |

Clinical-biological score prediction

|  | Accuracy | ROC-AUC | Score F1 |
|---|---|---|---|
| **With ERCF** | 81% | 71% | 89% |
| **Without ERCF** | 76% | 76% | 80% |

# FIG. 11

FIG. 12

# EUROPEAN SEARCH REPORT

**Application Number**

EP 24 30 7243

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/309909 A1 (HOLDER ANN [US] ET AL) 5 October 2023 (2023-10-05) * paragraphs [0004], [0005], [0008], [0039], [0057], [0058], [0166] – [0188], [0197] * ----- | 1-15 | INV. G16H50/20 G16H50/30 G16H50/70 G16H70/60 |

**TECHNICAL FIELDS SEARCHED    (IPC)**

G16H

The present search report has been drawn up for all claims

| Place of search | Date of completion of the search | Examiner |
|---|---|---|
| Munich | 5 May 2025 | Samulowitz, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 30 7243

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2025

| Patent document cited in search report | Publication date | Patent family member(s) | Publication date |
|---|---|---|---|
| US 2023309909 A1 | 05-10-2023 | US 2023309909 A1<br>WO 2023159191 A1 | 05-10-2023<br>24-08-2023 |

EPO FORM P0459

For more details about this annex : see Official Journal of the European Patent Office, No. 12/82